# EUROPEAN PATENT SPECIFICATION

(11) **EP 3 334 335 B1**
(45) Date of publication and mention of the grant of the patent: **02.06.2021**
(21) Application number: 16834674.0
(22) Date of filing: 11.08.2016
(51) Int. Cl.: A61B 5/0205, A61B 5/08

(54) **RESPIRATORY FUNCTION TESTING SYSTEM AND RESPIRATORY FUNCTION TESTING METHOD THEREOF**
ATEMFUNKTIONTESTSYSTEM UND ATEMFUNKTIONTESTVERFAHREN DAFÜR
SYSTÈME DE VÉRIFICATION DE LA FONCTION RESPIRATOIRE ET PROCÉDÉ DE VÉRIFICATION DE LA FONCTION RESPIRATOIRE ASSOCIÉ

(30) Priority: 12.08.2015 US 201562203951 P
(43) Date of publication of application: 20.06.2018
(73) Proprietor: Su, Chiachi, Taichung City 420, Taiwan (TW); Yen, Hsiaopao, New Taipei City 251, Taiwan (TW); Hsu, Peiling, Taipei City 111, Taiwan (TW); Chen, Chiahung, New Taipei City 241, Taiwan (TW)
(72) Inventor: Su, Chiachi, Taichung City 420, Taiwan (TW); Yen, Hsiaopao, New Taipei City 251, Taiwan (TW); Hsu, Peiling, Taipei City 111, Taiwan (TW); Chen, Chiahung, New Taipei City 241, Taiwan (TW)
(74) Representative: Patentanwaltskanzlei WILHELM & BECK
(86) International application number: PCT/CN2016/094621
(87) International publication number: WO 2017/025050

(56) References cited:
- EP-A1- 2 283 773
- EP-A1- 2 283 773
- WO-A1-2014/128331
- DE-B3-102004 055 967
- JP-A- 2014 064 675
- US-A- 4 602 644
- US-A1- 2012 271 188
- US-A1- 2013 190 641

## Description

### FIELD OF THE INVENTION

The present invention relates to respiratory function testing system and respiratory function testing method thereof, and more particularly to respiratory function testing system and respiratory function testing method thereof utilizing ultrasonic signals generated by exhaled air of a user.

### BACKGROUND OF THE INVENTION

Current common spirometry on the market is mainly plastic pressure indicatorbased or turbine based. For a spirometry with plastic pressure indicator based, the pressure generated by the exhalation blowing into the spirometry is for driving the sensor/receptor disposed at the end or side of the spirometry to generate a corresponding expiratory signal. This type of spirometry has an uncomplicated structure; however, it is impossible to continuously monitor the expiratory signal within one expiratory period. For a spirometry with turbine based, the pressure generated by the exhalation blowing into the spirometry is for driving the fan disposed in the spirometry to rotate. Through measuring the current generated by the rotating fans or using infrared technology, the cycles or speed of the rotations of the fans is counted; and therefore, data related to respiratory functions within one expiratory period is calculated based on the number or speed of the rotations of the fans. Document EP 2 283 773 A1 describes a system for processing a breathing signal. A whistling sound detection unit detects, in a breathing signal, a whistling sound generated by a whistle that is driven by a nasal air flow of a person; and a breathing phase determination unit determines a breathing phase based on the detected whistling sound. The JP 2014 064675 A describes an apnea detection system which can screen sleep apnea syndrome. The DE 10 2004 055 967 B3 describes an apparatus for determining a breathing parameter during an artificial respiration of a patient. The apparatus comprises a tube with integrated means for generating a sound signal. The US 2012/271188 A1 describes an apparatus for measuring levels of a specified gas in exhaled breath, the apparatus comprising a photoacoustic sensor for providing a measurement representative of the level of the specified gas in the exhaled air.

### SUMMARY OF THE INVENTION

One objective of the present invention is to provide a respiratory function testing system, wherein the respiratory function testing technical adopted by the respiratory function testing system is different from the spirometry mentioned in BACKGROUND OF THE INVENTION.

Another objective of the present invention is to provide a respiratory function testing method applicable to the respiratory function testing system.

The present invention provides a respiratory function testing system according to independent claim 1, which includes an air transforming device, a sound reception device and an operation device. The air transforming device is configured to collect exhaled air for a predetermined period and generate a full-range sound signal according to the collected exhaled air. The full-range sound signal at least contains an ultrasonic signal. The sound reception device is configured to receive and record the full-range sound signal. The operation device is in communication with the sound reception device and is configured to receive and compute the ultrasonic signal in the full-range sound signal recorded by the sound reception device to calculate respiratory function parameters.

The present invention provides a respiratory function testing method according to independent claim 4 applicable to the above respiratory function testing system. The respiratory function testing method includes: collecting exhaled air for a predetermined period and generating a full-range sound signal according to the collected exhaled air, wherein the full-range sound signal at least contains an ultrasonic signal; receiving and recording the full-range sound signal; and computing the ultrasonic signal in the full-range sound signal to generate corresponding respiratory function parameters.

In summary, by sequentially configuring the air transforming device to collect exhaled air for a predetermined period and generate a full-range sound signal according to the collected exhaled air, configuring the sound reception device to receive and record the full-range sound signal and configuring the operation device to receive and compute the ultrasonic signal contained in the full-range sound signal to generate a respiratory function parameter, the respiratory function testing system and the respiratory function testing method of the present invention can determine awhether a user has a normal respiratory function.

### BRIEF DESCRIPTION OF THE DRAWINGS

Other advantages, objectives and features of the present invention will become apparent from the following description referring to the attached drawings.
FIG. 1 is a schematic diagram of a respiratory function testing system in accordance with an embodiment of the present invention;
FIG. 2 is a schematic plot of full-range sound signal versus time in accordance with an embodiment of the present invention;
FIG. 3 is a schematic plot of sound pressure-time curve in accordance with an embodiment of the present invention;
FIG. 4 is a schematic plot of flow-time curve in accordance with an embodiment of the present invention;
FIG. 5 is a schematic plot of volume-time curve in accordance with an embodiment of the present invention; and
FIG. 6 is a flow chart of a respiratory function testing method in accordance with an embodiment of the present invention.

### DETAILED DESCRIPTION OF PREFERRED EMBODIMENTS

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

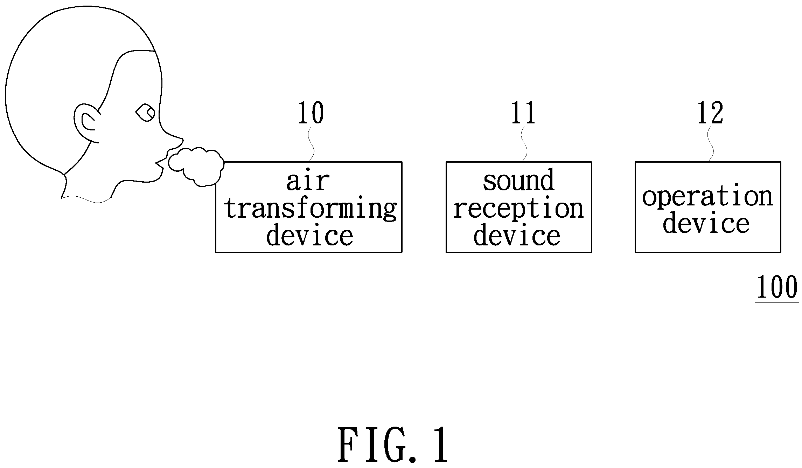

FIG. 1 is a schematic diagram of a respiratory function testing system in accordance with an embodiment of the present invention. As shown in FIG. 1, the respiratory function testing system 100 of the present embodiment includes an air transforming device 10, a sound reception device 11 and an operation device 12. The air transforming device 10 is configured to collect exhaled air for a predetermined period and generate a full-range sound signal according to the collected exhaled air, wherein the full-range sound signal at least contains an ultrasonic signal. In the present embodiment, the aforementioned predetermined period is, for example, the duration of a user continuously exhaling air toward the air transforming device 10. The sound reception device 11 is configured to receive and record the full-range sound signal generated by the air transforming device 10. In the present embodiment, the full-range sound signal generated by the air transforming device 10 according to the exhalation of the user covers all the frequency bands higher than 20 KHz; and specifically, the sound reception device 11 is configured to continuously receive and record the full-range sound signal at a frequency higher than 20 KHz for the predetermined period. Although different users may have different durations of exhalation due to the different respective respiratory functions, the respiratory function testing system 100 of the present invention is capable of testing the respiratory functions for the predetermined period for different users.

In the present embodiment, the sound reception device 11 is a microelectromechanical system (MEMS) or a microphone. Specifically, the sound reception device 11 is a highly-sensitive microphone capable of receiving and recording full-range sound signals and is selected from a group consisting of: omnidirectional microphone, cardioid microphone, hypercardioid microphone, shotgun microphone and bi-directional microphone. Because of having sensitive sound reception functions, each one of the microphones in the aforementioned group can be used to receive and record full-range sound signals and store the recorded full-range sound signal as an audio file, wherein the audio length of the audio file is the aforementioned predetermined period. The operation device 12 has a communicating connection with the sound reception device 11. The operation device 12 is configured to receive and compute the ultrasonic signal contained in the full-range sound signal recorded by the sound reception device 11 to generate a respiratory function parameter. In the present embodiment, the aforementioned communicating connection between the sound reception device 11 and the operation device 12 may be implemented via Bluetooth or Wi-Fi (wireless) means, though which the operation device 12 can receive the audio file stored by the sound reception device 11. In the present embodiment, the operation device 12 is an electronic device having computing capability such as smart phone or tablet, and the present invention is not limited thereto.

In one embodiment, the air transforming device 10 includes one or more silent whistles or Galton's whistles (not shown). The silent whistle or Galton's whistle is configured to generate the ultrasound signal according to the exhaled air while the user exhales air toward the air transforming device 10. The air transforming device 10 may include other types of ultrasound generator devices as long as such device is capable of generating the ultrasound signal according to the exhaled air of the user, and the present invention is not limited thereto.

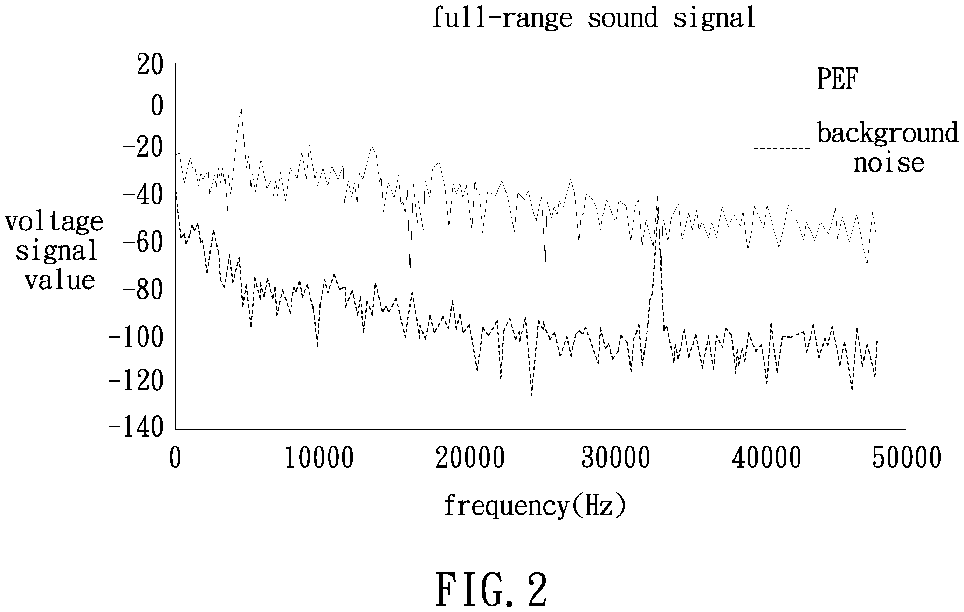

FIG. 2 is a schematic plot of full-range sound signal versus time in accordance with an embodiment of the present invention, wherein the full-range sound signal is represented by voltage signal values. After the operation device 12 receives the audio file of the full-range sound signal (FIG. 2) from the sound reception device 11, the user can select the full-range sound signal at a specific frequency for computing via an application program installed in the operation device 12. The purpose of the frequency selection is for reducing the interference from background noise in the environment and thereby increasing the accuracy of the computation. Specifically, the operation device 12 is configured to capture the sound pressure corresponding to the full-range sound signal at a predetermined frequency. The predetermined frequency may be determined by the application program automatically or set by the user. The aforementioned sound pressure is referred as the volume of the full-range sound signal and measured in decibels (dB) or fast Fourier transform (FFT). Full-range sound signal may have different sound pressures at different frequencies; therefore, by a proper frequency selection, a qualifying computing result may still be obtained if the user has a smaller amount of exhalation.

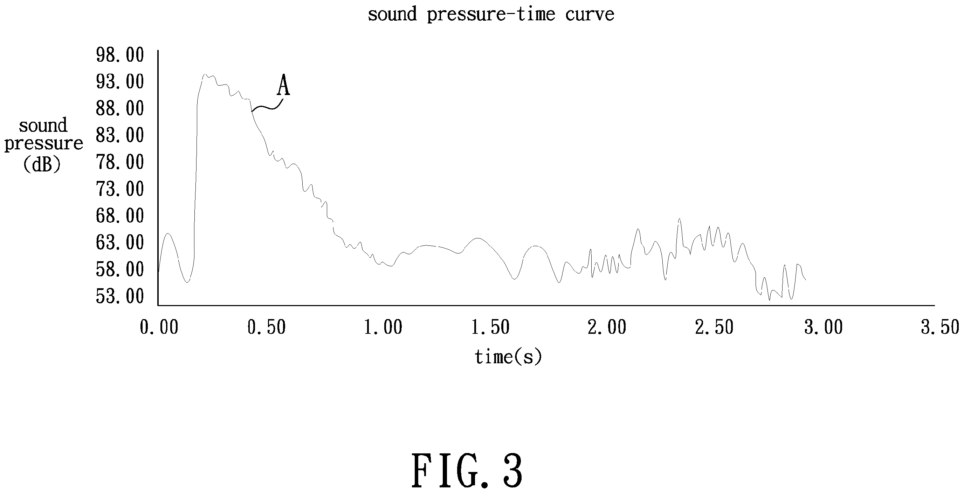

FIG. 3 is a schematic plot of sound pressure-time curve in accordance with an embodiment of the present invention. In FIG. 3, the curve A represents the sound pressures corresponding to the exhalation of the user within 0-3 seconds once a specific frequency is selected by the operation device 12; wherein the horizontal axis represents the dimension of time (e.g., in seconds) and the vertical axis represents the dimension of sound pressure (e.g., in dB). Specifically, the operation device 12 is configured to perform a transforming operation on the audio file of the ultrasonic signal contained in the full-range sound signal recorded by the sound reception device 11 to generate the respiratory function parameters. In the present embodiment, the respiratory function parameter includes peak expiratory flow (PEF), forced expiratory volume 1 (FEV1) and forced vital capacity (FVC).

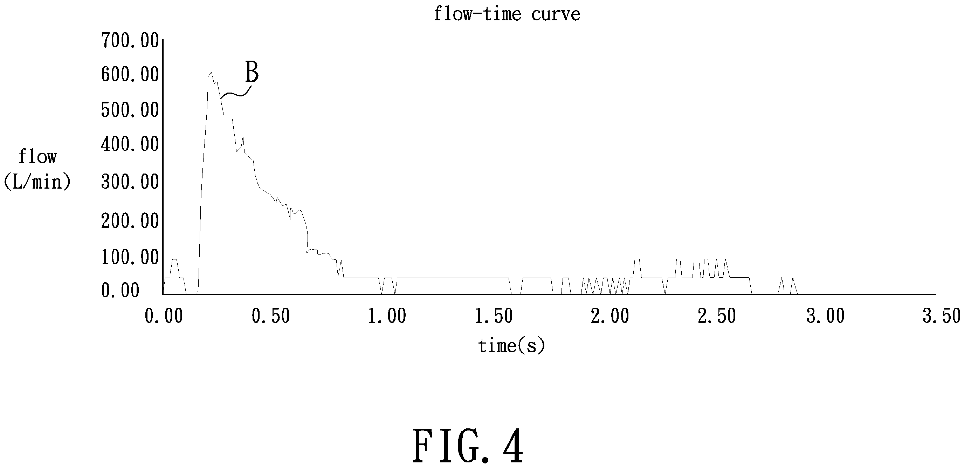

FIG. 4 is a schematic flow-time curve in accordance with the present invention, wherein the plot is transformed from FIG. 3 through a regression equation (e.g., a polynomial of one or more than one degrees such as y=ax+b, y=ax²+bx+c). Specifically, the curve B in FIG. 4 is obtained by comparing the curve A in FIG. 3 with the regression equation and verified with the spirometry certified by the US Food and Drug Administration (FDA). In the regression equation, y stands a testing value derived from a spirometry certified by FDA and x stands a testing value derived from the respiratory function testing system 100 of the present embodiment. In FIG. 4, PEF is referred as the maximum value of the curve B within a predetermined period; specifically, the PEF in FIG. 4 is 614.78 L/min and occurs at the point 0.22 in time.

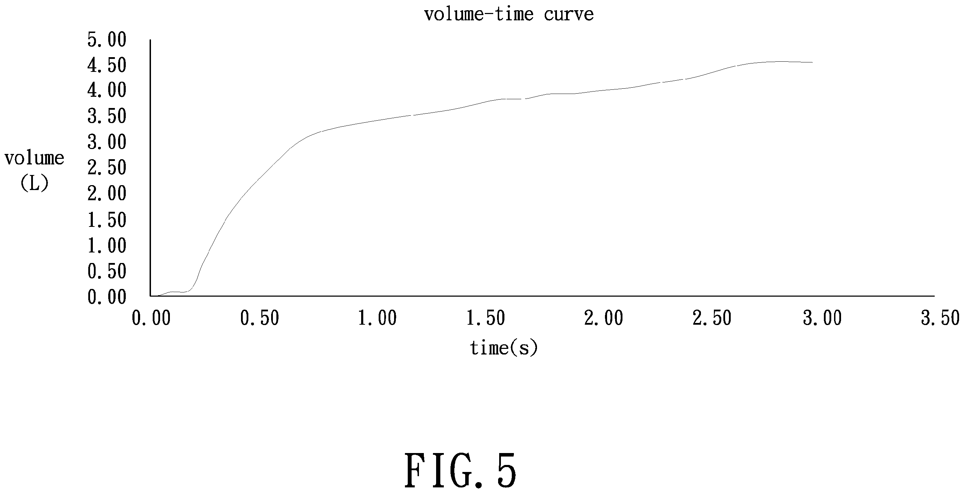

FIG. 5 is a schematic volume-time curve in accordance with an embodiment of the present invention, wherein the plot is transformed from FIG. 4. Specifically, the curve in FIG. 5 is obtained by performing the trapezoidal area integration formula on the curve B in FIG. 4 to accumulate the areas covered by the curve B. FEV1 is referred as the volume of expiration in the first second. In FIG. 5, for example, FEV1 is the value of volume corresponding to the point 1 in time. Further, FVC is referred as the volume of expiration within 0-3 seconds; that is, FVC is referred as the volume of expiration within a predetermined period of one respiratory function test. In FIG. 5, for example, FVC is the value of volume corresponding to the point 3 in time.

After all of the PEF, FEV1 and FVC are calculated, the condition of the respiratory functions of the user can be determined through comparing the calculated PEF, FEV1 and FVC with respective determined standard values. In general, the standard value of PEF is higher than 80% and the standard value of ratio of FVC to FEV1 (FVC/FEV1) is higher than 70%. Therefore, for an asthma patient, it is determined that the patient has a proper treatment if the variation (%) of PEF is lower than 20%; it is determined that the patient may need to increase the amount of medicine if the variation of PEF is in a range between 20%-30%; and it is determined that the patient is having asthma and may need an emergency treatment if the variation of PEF is higher than 30%. Herein the variation (%) of PEF is referred as: ((the maximum PEF)-(the minimum PEF))/((the maximum PEF)+(the minimum PEF))^{∗}100%.

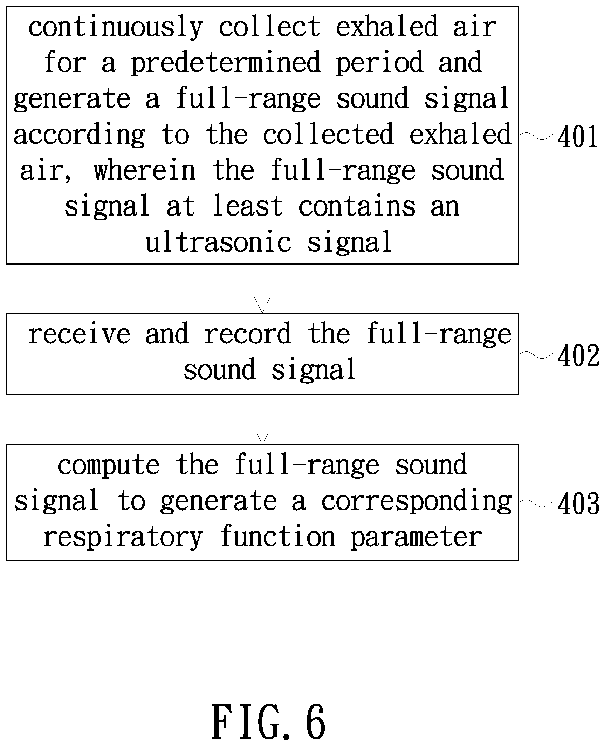

FIG. 6 is a flow chart of a respiratory function testing method in accordance with an embodiment of the present invention. As shown in FIG. 6, the respiratory function testing method of the present embodiment is applicable to the respiratory function testing system 100 and includes steps 401-403. Specifically, step 401: continuously collecting exhaled air for a predetermined period and generating a full-range sound signal according to the collected exhaled air, wherein the full-range sound signal at least contains an ultrasonic signal. Step 402: receiving and recording the full-range sound signal. Step 403: computing the full-range sound signal to generate a corresponding respiratory function parameter.

Refer to Table 1, which is a comparison between the PEF derived from the respiratory function testing system of the present invention and the PEF derived from the spirometry certified by FDA (hereunder is referred as a comparative example). As shown in Table 1, there are thirteen participants involved to the comparison; specifically, each one of the participants repeats the spirometric experiments three times for both of the systems of the present invention and the comparative example. The results of experiments indicate that all of the error rates of the system of the present invention relative to the comparative example are lower than 7%. Therefore, it is shown that the accuracy of the respiratory function testing system of the present invention is as good as that of the spirometry certified by FDA.

**Table 1: Comparison of PEF between the Present Invention and Comparative Examples**

| Participants | Gender | Age | Height (cm) | Comparative Examples PEF (L/min) | Present Invention PEF (L/min) | Error Rates |
|---|---|---|---|---|---|---|
| 1 | M | 23 | 167 | 619 | 657.75 | 6% |
| 2 | F | 10 | 140 | 286 | 277.68 | 3% |
| 3 | F | 30 | 159 | 341 | 346.88 | 2% |
| 4 | F | 28 | 153 | 375 | 355.99 | 5% |
| 5 | F | 28 | 153 | 356 | 344.20 | 3% |
| 6 | M | 40 | 176 | 618 | 614.91 | 1% |
| 7 | F | 46 | 156 | 367 | 364.68 | 1% |
| 8 | F | 28 | 153 | 366 | 356.29 | 3% |
| 9 | F | 28 | 153 | 380 | 361.81 | 5% |
| 10 | M | 40 | 176 | 622 | 615.76 | 1% |
| 11 | M | 58 | 168 | 545 | 554.91 | 2% |
| 12 | M | 40 | 176 | 588 | 629.67 | 7% |
| 13 | F | 28 | 153 | 365 | 374.77 | 3% |

In summary, by sequentially configuring the air transforming device to collect exhaled air for a predetermined period and generate a full-range sound signal according to the collected exhaled air, configuring the sound reception device to receive and record the full-range sound signal and configuring the operation device to receive and compute the ultrasonic signal contained in the full-range sound signal to generate a respiratory function parameter, the respiratory function testing system and the respiratory function testing method of the present invention can determine whether a user has a normal respiratory function.

While the invention has been described in terms of what is presently considered to be the most practical and preferred embodiments, it is to be understood that the invention needs not be limited to the disclosed embodiments. On the contrary, it is intended to cover various modifications and similar arrangements included within the scope of the appended claims which are to be accorded with the broadest interpretation so as to encompass all such modifications and similar structures. The present invention is limited by the scope of the appended claims.

## Claims

1. A respiratory function testing system (100)
**characterized by**:
an air transforming device (10), configured to collect exhaled air for a predetermined period and generate a full-range sound signal according to the collected exhaled air, wherein the full-range sound signal comprises an ultrasonic signal;
a sound reception device (11), configured to receive and record the full-range sound signal; and
an operation device (12), communicating with the sound reception device (11) and configured to receive and compute the ultrasonic signal in the full-range sound signal recorded by the sound reception device (11) to generate a respiratory function parameter comprising peak expiratory flow (PEF), forced expiratory volume 1 (FEV1) and forced vital capacity (FVC),
wherein the operation device (12) is further configured to capture the sound pressure corresponding to the full-range sound signal at a predetermined frequency and the sound pressure corresponding to the predetermined frequency is adapted for reducing interference from a background noise,
**characterised in that** to compute the ultrasonic signal comprises transforming a sound pressure of the ultrasonic signal under the predetermined period into a flow of the exhaled air under the predetermined period through a regression equation.

2. The respiratory function testing system (100) according to claim 1, wherein the air transforming device (10) comprises a silent whistle or a Galton's whistle.

3. The respiratory function testing system (100) according to claim 1, wherein the sound reception device (11) comprises a microelectromechanical system or a microphone, and the microphone is selected from a group consisting of: an omnidirectional microphone, a cardioid microphone, a hypercardioid microphone, a shotgun microphone and a bi-directional microphone.

4. A respiratory function testing method of a respiratory function testing system (100), the respiratory function testing system (100) comprising an air transforming device (10), a sound reception device (11) and an operation device (12), and the respiratory function testing method comprising:
collecting exhaled air for a predetermined period and generating a full-range sound signal according to the collected exhaled air, wherein the full-range sound signal comprises an ultrasonic signal (401);
receiving and recording the full-range sound signal (402); and
capturing the sound pressure corresponding to the full-range sound signal at a predetermined frequency;
computing the ultrasonic signal in the full-range sound signal to generate a corresponding respiratory function parameter comprising peak expiratory flow (PEF), forced expiratory volume 1 (FEV1) and forced vital capacity (FVC),
**characterised in that** to compute the ultrasonic signal comprises transforming a sound pressure of the ultrasonic signal under the predetermined period into a flow of the exhaled air under the predetermined period through a regression equation (403).

5. The respiratory function testing method according to claim 4, wherein the air transforming device (10) comprises a silent whistle or a Galton's whistle, and the silent whistle or the Galton's whistle is configured to generate the full-range sound signal according to the collected exhaled air.

6. The respiratory function testing method according to claim 4, wherein the sound reception device (11) comprises a microelectromechanical system or a microphone and the microphone is configured to receive and record the full-range sound signal.

## Patentansprüche

1. Atemfunktionstestsystem (100), **gekennzeichnet durch**:
eine Lufttransformationsvorrichtung (10), die ausgestaltet ist, um für einen vorbestimmten Zeitraum ausgeatmete Luft zu sammeln und ein Vollbereichschallsignal gemäß der gesammelten ausgeatmeten Luft zu generieren, wobei das Vollbereichschallsignal ein Ultraschallsignal umfasst;
eine Schallempfangsvorrichtung (11), die ausgestaltet ist, um das Vollbereichschallsignal zu empfangen und aufzuzeichnen; und
eine Betriebsvorrichtung (12), die mit der Schallempfangsvorrichtung (11) kommuniziert und ausgestaltet ist, um das Ultraschallsignal in dem Vollbereichsschallsignal, das von der Schallempfangsvorrichtung (11) aufgezeichnet wurde, zu empfangen und zu berechnen, um einen Atemfunktionsparameter zu generieren, der exspiratorischen Spitzenfluss (Peak Flow, PEF), Einsekundenkapazität (FEV1) und forcierte Vitalkapazität (FVC) umfasst,
wobei die Betriebsvorrichtung (12) des Weiteren ausgestaltet ist, um den Schalldruck entsprechend dem Vollbereichschallsignal bei einer vorbestimmten Frequenz zu erfassen, und der Schalldruck entsprechend der vorbestimmten Frequenz zum Reduzieren der Interferenz durch Hintergrundrauschen adaptiert wird,
**dadurch gekennzeichnet, dass**
Berechnen des Ultraschallsignals Transformieren eines Schalldrucks des Ultraschallsignals unter dem vorbestimmten Zeitraum in einen Fluss der ausgeatmeten Luft unter dem vorbestimmten Zeitraum durch eine Regressionsgleichung umfasst.

2. Atemfunktionstestsystem (100) nach Anspruch 1, wobei die Lufttransformationsvorrichtung (10) eine Lautlospfeife oder eine Galton-Pfeife umfasst.

3. Atemfunktionstestsystem (100) nach Anspruch 1, wobei die Schallempfangsvorrichtung (11) ein mikroelektromechanisches System oder ein Mikrofon umfasst und das Mikrofon ausgewählt ist aus der Gruppe bestehend aus einem omnidirektionalen Mikrofon, einem Kardioidmikrofon, einem Hyperkardioidmikrofon, einem Richtrohrmikrofon und einem bidirektionalen Mikrofon.

4. Atemfunktionstestverfahren eines Atemfunktionstestsystems (100), wobei das Atemfunktionstestsystem (100) eine Lufttransformationsvorrichtung (10), eine Schallempfangsvorrichtung (11) und eine Betriebsvorrichtung (12) umfasst, und wobei das Atemfunktionstestverfahren umfasst:
Sammeln von ausgeatmeter Luft für einen vorbestimmten Zeitraum und Generieren eines Vollbereichschallsignals gemäß der gesammelten ausgeatmeten Luft, wobei das Vollbereichschallsignal ein Ultraschallsignal (401) umfasst;
Empfangen und Aufzeichnen des Vollbereichschallsignals (402); und
Erfassen des Schalldrucks entsprechend dem Vollbereichschallsignal bei einer vorbestimmten Frequenz;
Berechnen des Ultraschallsignals in dem Vollbereichschallsignal, um einen entsprechenden Atemfunktionsparameter zu generieren, der Peak Flow (PEF), Einsekundenkapazität (FEV1) und forcierte Vitalkapazität (FVC) umfasst,
**dadurch gekennzeichnet, dass**
Berechnen des Ultraschallsignals Transformieren eines Schalldrucks des Ultraschallsignals unter dem vorbestimmten Zeitraum in einen Fluss der ausgeatmeten Luft unter dem vorbestimmten Zeitraum durch eine Regressionsgleichung (403) umfasst.

5. Atemfunktionstestverfahren nach Anspruch 4, wobei die Lufttransformationsvorrichtung (10) eine Lautlospfeife oder Galton-Pfeife umfasst, und die Lautlospfeife oder Galton-Pfeife ausgestaltet ist, um das Vollbereichschallsignal gemäß der gesammelten ausgeatmeten Luft zu generieren.

6. Atemfunktionstestverfahren nach Anspruch 4, wobei die Schallempfangsvorrichtung (11) ein mikroelektromechanisches System oder ein Mikrofon umfasst und das Mikrofon ausgestaltet ist, um das Vollbereichschallsignal zu empfangen und aufzuzeichnen.

## Revendications

1. Système de vérification de la fonction respiratoire (100) **caractérisé par** :
un dispositif de transformation d'air (10), configuré pour recueillir l'air expiré pendant une période de temps prédéterminée et générer un signal sonore pleine gamme en fonction de l'air expiré recueilli, le signal sonore pleine gamme comprenant un signal ultrasonore ;
un dispositif de réception de sons (11), configuré pour recevoir et enregistrer le signal sonore pleine gamme ; et
un dispositif d'actionnement (12), communiquant avec le dispositif de réception de sons (11) et configuré pour recevoir et calculer le signal ultrasonore dans le signal sonore pleine gamme enregistré par le dispositif de réception de sons (11) pour générer un paramètre de fonction respiratoire comprenant un débit expiratoire de pointe (PEP), un volume expiratoire forcé (FEV1) et une capacité vitale forcée (FVC),
le dispositif d'actionnement (12) étant en outre configuré pour capturer la pression sonore correspondant au signal sonore pleine gamme à une fréquence prédéterminée et la pression sonore correspondant à la fréquence prédéterminée étant adaptée pour réduire l'interférence d'un bruit de fond,
**caractérisé en ce que**
le calcul du signal ultrasonore comprend la transformation d'une pression acoustique du signal ultrasonore pendant la période de temps prédéterminée en un débit de l'air expiré pendant la période de temps prédéterminée par une équation de régression.

2. Système de vérification de la fonction respiratoire (100) selon la revendication 1, le dispositif de transformation d'air (10) comprenant un sifflet silencieux ou un sifflet de Galton.

3. Système de vérification de la fonction respiratoire (100) selon la revendication 1, le dispositif de réception de sons (11) comprenant un système microélectromécanique ou un microphone, et le microphone étant choisi dans un groupe constitué par : un microphone omnidirectionnel, un microphone cardioïde, un microphone hypercardioïde, un microphone canon et un microphone bidirectionnel.

4. Procédé de vérification de la fonction respiratoire d'un système de vérification de la fonction respiratoire (100), le système de vérification de la fonction respiratoire (100) comprenant un dispositif de transformation d'air (10), un dispositif de réception de sons (11) et un dispositif d'actionnement (12), et le procédé de vérification de la fonction respiratoire comprenant :
le recueil de l'air expiré pendant une période de temps prédéterminée et la génération d'un signal sonore pleine gamme en fonction de l'air expiré recueilli, le signal sonore pleine gamme comprenant un signal ultrasonore (401) ;
la réception et l'enregistrement du signal sonore pleine gamme (402) ; et
la capture de la pression acoustique correspondant au signal sonore pleine gamme à une fréquence prédéterminée ;
le calcul du signal ultrasonore dans le signal sonore pleine gamme pour générer un paramètre de fonction respiratoire correspondant comprenant le débit expiratoire de pointe (PEP), le volume expiratoire forcé 1 (FEV1) et la capacité vitale forcée (FVC),
**caractérisé en ce que**
le calcul du signal ultrasonore comprend la transformation d'une pression acoustique du signal ultrasonore pendant la période de temps prédéterminée en un débit de l'air expiré pendant la période de temps prédéterminée par une équation de régression (403).

5. Procédé de vérification de la fonction respiratoire selon la revendication 4, le dispositif de transformation d'air (10) comprenant un sifflet silencieux ou un sifflet de Galton, et le sifflet silencieux ou le sifflet de Galton étant configuré pour générer le signal sonore pleine gamme en fonction de l'air expiré recueilli.

6. Procédé de vérification de la fonction respiratoire selon la revendication 4, le dispositif de réception de sons (11) comprenant un système microélectromécanique ou un microphone et le microphone étant configuré pour recevoir et enregistrer le signal sonore pleine gamme.
